# EUROPÄISCHE PATENTSCHRIFT

(11) **EP 2 484 776 B1**
(45) Veröffentlichungstag und Bekanntmachung des Hinweises auf die Patenterteilung: **17.12.2014**
(21) Anmeldenummer: 12152034.0
(22) Anmeldetag: 23.01.2012
(51) Int. Cl.: C12Q 1/56, G01N 33/86

(54) **Heparin-insensitives Verfahren zur Bestimmung von direkten Gerinnungsfaktorinhibitoren**
Heparin-insensitive method for determination of direct clotting factor inhibitors
Procédé héparine-insensible pour la détection des inhibiteurs directes du facteur de coagulation

(30) Priorität: 07.02.2011 EP 11153524
(43) Veröffentlichungstag der Anmeldung: 08.08.2012
(73) Patentinhaber: Siemens Healthcare Diagnostics Products GmbH, 35041 Marburg (DE)
(72) Erfinder: Braun, Konrad, 35085 Ebsdorfergrund (DE); Klein, Wolfgang, 35043 Marburg (DE); Zander, Norbert, 35039 Marburg (DE); Timme, Michael, 35091 Coelbe (DE)

(56) Entgegenhaltungen:
- EP-A2- 0 297 597
- WO-A1-92/17187
- SCHWIENHORST A: "Direct thrombin inhibitors - a survey of recent developments", CMLS CELLULAR AND MOLECULAR LIFE SCIENCES, BIRKHÄUSER-VERLAG, BA, Bd. 63, Nr. 23, 13. November 2006 (2006-11-13), Seiten 2773-2791, XP019457694, ISSN: 1420-9071, DOI: DOI:10.1007/S00018-006-6219-Z

## Beschreibung

Die vorliegende Erfindung liegt auf dem Gebiet der Gerinnungsdiagnostik und betrifft ein Heparin-insensitives Verfahren zur Bestimmung von direkten Gerinnungsfaktorinhibitoren in einer Probe, insbesondere von direkten Thrombin- und Faktor Xa-Inhibitoren, sowie ein Testkit zur Verwendung in einem solchen Verfahren.

In der Antikoagulationstherapie werden zunehmend neue direkte Gerinnungsinhibitoren, insbesondere direkte Thrombin-(Faktor IIa-) und Faktor Xa-Inhibitoren eingesetzt. Diese neuen Gerinnungshemmer haben das Potenzial, die bislang benutzten indirekten Gerinnungshemmer, vor allem Heparin und dessen Derivate, die ihre gerinnungshemmende Wirkung nur im Zusammenspiel mit Kofaktoren wie Antithrombin oder Heparin Cofaktor II entfalten, abzulösen. Derzeit kommen jedoch weiterhin vornehmlich die verschiedenen Heparine zum Einsatz.

Die gerinnungshemmende Wirkung aller Heparine beruht auf ihrer Komplexbildung mit Antithrombin (AT, Antithrombin III), dem wichtigsten plasmatischen Inhibitor aktivierter Gerinnungsfaktoren. Antithrombin gehört zur Gruppe der Serinproteaseinihibitoren (Serpine) und hemmt die Gerinnungsfaktoren Thrombin (Faktor IIa, FIIa) und Faktor Xa (FXa) sowie in geringem Ausmaß auch die anderen Serinproteasen FIXa, FXIa, FXIIa, Kallikrein und Plasmin. Durch die Bindung von Heparin an Antithrombin kommt es zu einer Konformationsänderung des Antithrombins, welche die inhibitorische Wirkung des Antithrombins um ein Vielfaches verstärkt. Die Bindungsstelle in Heparinmolekülen, die für die Bindung an Antithrombin verantwortlich ist, besteht aus einer charakteristischen Pentasaccharidsequenz. Eine vollständig synthetische Form dieses Pentasaccharids (Fondaparinux) wird ebenso wie UFH oder LMWH zur medikamentösen Hemmung der Gerinnungsfähigkeit eingesetzt.

Unfraktionierte Heparine (UFH) und fraktionierte Heparine, Heparinderivate, Heparinoide bzw. Pentasaccharide unterscheiden sich in ihrer antikoagulatorischen Wirkung. Während UFH Thrombin und Faktor Xa gleichermaßen hemmen, weisen LMWH überwiegend eine Faktor Xa-hemmende Wirkung und nur in geringerem Maße eine Thrombin-hemmende Wirkung auf. Pentasaccharide wie Fondaparinux hemmen selektiv Faktor Xa und zeigen gar keine Thrombinhemmung.

Bei gewissen Krankheitsbildern, wie z.B. im Falle einer Heparin-induzierten Thrombozytopenie (HIT), muss jedoch die Behandlung mit Heparin abgebrochen werden, und es müssen dem Patienten andere Antikoagulanzien, bevorzugt direkte Thrombin- und/oder Faktor Xa-Inhibitoren, verabreicht werden. Bei solchen Patienten ist es notwendig, über diagnostische Methoden zu verfügen, die es ermöglichen, die Thrombin- bzw. Faktor Xa-Hemmung durch die direkten Inhibitoren unabhängig von der Thrombin- bzw. Faktor Xa-Hemmung durch etwaige indirekte Inhibitoren, wie z.B. Heparin, zu bestimmen.

Die Bestimmung von Thrombin- bzw. Faktor Xa-Inhibitoren erfolgt überlicherweise mit Hilfe chromogener Testverfahren. Bei diesen Verfahren wird die Patientenprobe, die einen Thrombin- bzw. Faktor Xa-Inhibitor enthält, mit einer definierten Menge des entsprechenden aktivierten Gerinnungsfaktors und einem chromogenen Substrat für den aktivierten Gerinnungsfaktor vermischt, und die im Reaktionsansatz verbleibende Aktivität des Gerinnungsfaktors wird photometrisch gemessen. Je höher die Konzentration des Inhibitors in der Patientenprobe ist, desto mehr wird die Aktivität des zugegebenen Gerinnungsfaktors gehemmt und desto geringer ist die gemessene Aktivität im Reaktionsansatz. Beispielsweise in EP 0034320 B1 oder in EP 0004271 A2 sind derartige Thrombin- bzw. Faktor Xa-basierte, chromogene Testverfahren beschrieben.

Nachteilig bei diesem Testprinzip ist jedoch, dass jede Art von Thrombin- bzw. Faktor Xa-inhibierender Aktivität gemessen wird, ohne dass differenziert werden kann, ob bzw. zu welchem Anteil die inhibierende Aktivität von indirekten oder direkten Thrombin- bzw. Faktor Xa-Inhibitoren herrührt.

Im Stand der Technik wird dieses Problem dadurch gelöst, dass bei Testen, in denen andere Thrombin- bzw. Faktor Xa-Inhibitoren als Heparin gemessen werden sollen, die inhibierende Aktivität des Heparins neutralisiert oder ausgeblendet wird.

In einem ersten bekannten Verfahren werden etwaige in der Probe enthaltene Heparine durch die Zugabe von Heparinabbauenden Enzymen, wie z.B. von Heparinase, zur Patientenprobe degradiert. Durch die Heparinaseaktivität werden Heparin und alle Heparinderivate, die eine Glucosaminoglucansequenz aufweisen, enzymatisch abgebaut, wodurch die indirekte, Antithrombin-vermittelte, inhibitorische Aktivität von Heparin eliminiert wird. Andere, direkte Thrombin- bzw. Faktor Xa-Inhibitoren, die keine Glucosaminoglucansequenz aufweisen, sind nach einer solchen Vorbehandlung der Probe quantifizierbar. Dieses Prinzip ist in dem US-Patent 5,262,325 beschrieben.

Bei einem anderen bekannten Verfahren zur Bestimmung von direkten Faktor Xa-Inhibitoren in Gegenwart von Heparin wird ein Polyethylenglykol-konjugierter Faktor Xa zur Probe gegeben und dessen Hemmung mit Hilfe eines chromogenen Substrats bestimmt. Polyethylenglykol-konjugierter Faktor Xa ist zwar durch direkte FXa-Inhibitoren, wie z.B. Rivaroxaban, jedoch nicht durch Antithrombin-Heparin Komplexe inhibierbar. Die Verwendung von modifiziertem Faktor Xa ermöglicht daher die spezifische Bestimmung von direkten Faktor Xa-Inhibitoren in Gegenwart von Heparin (Posterabstract P17-05, Lange, U. et al. , A simple and specific assay for direct factor Xa inhibitors in plasma without interference by heparins. Kongressausgabe Hämostaseologie 1/2010).

Bei einem weiteren bekannten Verfahren zur Bestimmung von direkten Faktor Xa-Inhibitoren in Gegenwart von Heparin wird Faktor Xa und dessen Hemmung mit Hilfe eines chromogenen Substrats und in Gegenwart von chaotropen Substanzen bestimmt. Die chaotropen Substanzen verhindern offensichtlich die Antithrombin-Heparin-Interaktion, so dass auch dieses Verfahren insensitiv für die Faktor Xa-inhibierende Wirkung von Heparinen ist und sich daher für die spezifische Bestimmung von direkten Faktor Xa-Inhibitoren, wie z.B. Rivaroxaban eignet (Posterabstract P01-17, Samama, M.M. et al., Specific and rapid measurement of rivaroxaban using a new, dedicated chromogenic assay. Kongressausgabe Hämostaseologie 1/2010).

Weitere bekannte Methoden zur Neutralisierung von Heparin in Patientenproben umfassen die Zugabe von Polykationen, wie Hexadimethrinbromid (Polybrene®) oder von Metallsalzen, wie Kupfer- oder Zinksalzen, deren Ionen mit Heparin einen Komplex bilden (EP 0697463 A1).

EP 0 297 597 A2 enthüllt bereits ein Verfahren zum Bestimmen der Aktivität von Serinprotease-Inhibitoren. Das Dokument offenbart nicht, dass der Reaktionsansatz mit einem das Oxidationsmittel neutralisierenden Agens vermischt wird, bevor eine definierte Menge Thrombin zum Reaktionsansatz gegeben wird.

### Zusammenfassung der Erfindung

Die Erfindung in ihrer breitesten Form ist im unabhängigen Anspruch 1 definiert:

Verfahren zur Bestimmung eines direkten Inhibitors eines Gerinnungsfaktors in einer Probe, wobei der direkte Inhibitor des Gerinnungsfaktors ausgewählt ist aus der Gruppe der direkten Thrombininhibitoren Hirudin, Dabigatran, Melagatran, Argatroban, Ximelagatran, Bivalirudin, Lepirudin, MCC-977,SSR-182289, TGN-255, TGN-167, ARC-183 und Odiparcil oder aus der Gruppe der direkten Faktor Xa-Inhibitoren Rivaroxaban, Apixaban, Otamixaban, LY 517717, YM 153, DU-176b, DX-9065a und KFA-1982, wobei das Verfahren folgende Schritte in der genannten Reihenfolge aufweiset:
a) Vermischen der Probe mit einem Oxidationsmittel zu einem Reaktionsansatz;
b) Inkubation des Reaktionsansatzes;
c) Vermischen des Reaktionsansatzes mit einem das Oxidationsmittel neutralisierenden Agens;
d) Zugabe einer definierten Menge des aktivierten Gerinnungsfaktors Thrombin oder Faktor Xa zum Reaktionsansatz;
e) Bestimmung der Hemmung des zugesetzten aktivierten Gerinnungsfaktors.

Bevorzugte Ausführungsformen finden sich in den abhängigen Ansprüchen 2-5.

### Detaillierte Beschreibung

Der vorliegenden Erfindung lag die Aufgabe zugrunde, ein weiteres Verfahren zur spezifischen Bestimmung von Gerinnungsfaktorinhibitoren bereit zu stellen, welches insensitiv ist gegenüber Heparin und dessen Derivaten und das leicht auf einem Gerinnungsmesssystem automatisiert werden kann.

Die Aufgabe wird dadurch gelöst, dass eine Probe, die vermutlich Heparin und einen oder mehrere direkte Gerinnungsinhibitoren enthält, zunächst mit einem Oxidationsmittel zu einem Reaktionsansatz vermischt und inkubiert wird und anschließend der Reaktionsansatz mit einem das Oxidationsmittel neutralisierenden Agens vermischt wird. Dies bewirkt, dass Antithrombin-Heparin-Interaktionen verhindert werden und so die Thrombin- und die Faktor Xa-inhibierende Aktivität von Heparin eliminiert wird, wodurch bei der nachfolgenden Bestimmung der Hemmung eines zugegebenen aktivierten Gerinnungsfaktors, wie Thrombin oder Faktor Xa, spezifisch nur der inhibierende Einfluss direkter Gerinnungsinhibitoren gemessen wird gemäß Anspruch 1.

Die vorliegende Erfindung betrifft also ein Verfahren zur Bestimmung eines direkten Inhibitors eines Gerinnungsfaktors in einer Probe, wobei das Verfahren folgende Schritte aufweist:
a) Vermischen der Probe mit einem Oxidationsmittel zu einem Reaktionsansatz;
b) Inkubation des Reaktionsansatzes;
c) Vermischen des Reaktionsansatzes mit einem das Oxidationsmittel neutralisierenden Agens;
d) Zugabe einer definierten Menge des aktivierten Gerinnungsfaktors zum Reaktionsansatz;
e) Bestimmung der Hemmung des zugesetzten aktivierten Gerinnungsfaktors gemäß Anspruch 1.

Der Begriff "direkter Inhibitor eines Gerinnungsfaktors" oder "direktes Antikoagulanz" betrifft nicht-physiologische, bevorzugterweise therapeutisch wirksame Substanzen, die durch direkte Interaktion mit dem Gerinnungsfaktor die Aktivität des Gerinnungsfaktors reduzieren. Direkte Inhibitoren von Gerinnungsfaktoren, zu deren Bestimmung das erfindungsgemäße Verfahren geeignet ist, sind inbesondere direkte Inhibitoren des Gerinnungsfaktors Thrombin, wie z.B. Hirudin, Dabigatran, Melagatran, Argatroban, Ximelagatran, Bivalirudin, Lepirudin, MCC-977, SSR-182289, TGN-255, TGN-167, ARC-183 und Odiparcil oder direkte Inhibitoren des Gerinnungsfaktors Faktor Xa, wie z.B. Rivaroxaban, Apixaban, Otamixaban (welche in der neuen Wirkstoffklasse der Xabane zusammengefasst werden),LY 517717, YM 153, DU-176b, DX-9065a und KFA-1982.

Direkte Inhibitoren sind zu unterscheiden von "indirekten Inhibitoren" oder "indirekten Antikoagulanzien", welche nur durch die Interaktion mit physiologischen Kofaktoren, wie z.B. Antithrombin oder Heparin Cofaktor II, die Aktivität eines Gerinnungsfaktors reduzieren. Indirekte Inhibitoren von Gerinnungsfaktoren, für die das erfindungsgemäße Verfahren insensitiv ist, sind inbesondere Heparine und Heparin-ähnliche Substanzen, die mindestens eine Glucosaminoglucansequenz aufweisen, wie z.B. unfraktionierte, hochmolekulare Heparine (HMWH, UFH), fraktionierte, niedermolekulare Heparine (LMWH), Heparinderivate und Heparinoide. Heparinderivate und Heparinoide sind enzymatisch und/oder chemisch veränderte Glukosaminoglykanketten, deren antikoagulatorische Eigenschaften durch die gezielte Beeinflussung von Glykankettenlänge, Sulfatierungs- bzw. Acetylierungsmuster oder die Mischung unterschiedlicher Glukosaminoglykane biotechnologisch modifiziert werden können, wie z.B. bei Danaparoid-Natrium, einem Gemisch aus Glykosaminoglykanen, das überwiegend aus Heparansulfat und zu einem geringeren Anteil aus Dermatansulfat und Chondroitinsulfat besteht. Auch synthetische Heparine, wie das Pentasaccharid Fondaparinux wirken indirekt über Antithrombin. In Sinne der vorliegenden Erfindung umfasst der Begriff "Heparin" im Folgenden alle vorgenannten Heparine, Heparinoide und Heparinderivate.

Der Begriff "Oxidationsmittel" umfasst Substanzen, welche die Oxidation sterisch relevanter Aminosäuren von plasmatischen Serinprotease-Inhibitoren, insbesondere von Antithrombin, bewirken. Durch die Oxidation sterisch relevanter Aminosäuren wird die Ausbildung der aktiven Konformation des Serinprotease-Inhibitors verhindert und damit die Inhibierung des aktiven Zentrums von Serinproteasen wie Faktor Xa oder Faktor IIa. Bevorzugte Oxidationsmittel sind z.B. Hypochloride und deren Salze, beispielsweise Natrium- oder Kaliumhypochlorit, welche im neutralen pH-Bereich aktiv sind. Dies hat den Vorteil, dass die zu untersuchenden Analyten, die direkten Gerinnungsfaktor-Inhibitoren, nicht durch extreme pH-Werte im Reaktionsansatz degradiert werden. Häufig ist Methionin an der Ausbildung sterisch relevanter Konformationen in Serinprotease-Inhibitoren beteiligt. Weitere bevorzugte Oxidationsmittel sind daher Substanzen aus der Gruppe der reaktiven Halogene, Wasserstoffperoxide, Peroxomonoschwefelsäure, Peroxodischwefelsäure, Chloramin B, Chloramin T, Hypochlorsäure, N-Chlorosuccinemid oder Salze der vorab aufgeführten Substanzen, welche Methionin zu Methioninsulfoxid oxidieren und somit die Serinprotease-Inhibitor-Aktivität unterbinden (siehe auch Shechter, Y. et. al., Selective oxidation of methionine residues in proteins. Biochemistry 1975, 14(20), 4497-4503).

Der Begriff "das Oxidationsmittel neutralisierendes Agens" umfasst Substanzen, welche die oxidative Aktivität des eingesetzten Oxidationsmittels neutralisieren, also aufheben, so dass von diesem keine weiteren, gegebenenfalls das Messprinzip beeinflussende Störungen ausgehen können. Ein geeignetes die oxidative Aktivität des Oxidationsmittels neutralisierendes Agens ist ein mildes Reduktionsmittel. Im einfachsten Fall genügt hierzu eine Halogensäure, bevorzugt niedermolare, wässrige Lösungen von Chlorwasserstoffsäuren, wie z.B. Salzsäure, welche das Oxidationsmittel destabilisiert, so dass beide Komponenten im wässrigen System vollständig abreagieren können. Alternativ können auch Fruchtzucker, Polyole, Fruchtsäuren und deren Salze benutzt werden. Nicht geeignet sind alle stärkeren Reduktionsmittel, wie zum Beispiel Borsäure, welche die nachfolgenden Reaktionsschritte stören könnten.

Unter einer "Probe" ist im Sinne der Erfindung das Material zu verstehen, das den zu bestimmenden direkten Gerinnungsinhibitor vermutlich enthält. Der Begriff Probe umfasst insbesondere menschliche oder tierische Körperflüssigkeiten, vornehmlich Blut, Plasma und Serum.

Das erfindungsgemäße Verfahren zur Bestimmung eines direkten Inhibitors eines Gerinnungsfaktors umfasst unter anderem das Vermischen der Probe mit einem Oxidationsmittel zu einem Reaktionsansatz, welcher dann für einen begrenzten Zeitraum inkubiert wird, bevor dem Reaktionsansatz ein das Oxidationsmittel neutralisierendes Agens zugegeben wird.

Die Inkubationsdauer des Reaktionsansatzes nach Zugabe des Oxidationsmittels sollte mindestens 30 Sekunden betragen, bevor das neutralisierende Agens zugegeben wird. Bevorzugterweise wird der Reaktionsansatz für einen Zeitraum von etwa 30 bis 180 Sekunden, besonders bevorzugt für einen Zeitraum von 90 bis 120 Sekunden inkubiert.

Die so vorbehandelte Probe wird dann einem konventionellen Gerinnungsfaktoraktivitätstest unterworfen, wobei der erfindungsgemäß vorbehandelten Probe eine definierte Menge desjenigen aktivierten Gerinnungsfaktors zugegeben wird, für welchen der zu bestimmende direkte Gerinnungsinhibitor spezifisch ist. Bei einem Verfahren zur Bestimmung eines direkten Thrombininhibitors wird konsequenterweise eine definierte Menge Thrombin zugegeben; bei einem Verfahren zur Bestimmung eines direkten Faktor Xa-Inhibitors wird eine definierte Menge Faktor Xa zugegeben, und die Inhibitor-abhängige Inaktivierung der Aktivität des zugegebenen Gerinnungsfaktors wird gemessen. Bevorzugterweise wird die Inhibitor-abhängige Inaktivierung der amidolytischen Aktivität des zugegebenen Gerinnungsfaktors mit Hilfe eines chromogenen, fluorogenen oder anderweitig markierten Substrats ermittelt, das spezifisch von dem aktivierten Gerinnungsfaktor gespalten wird. Bei der abspaltbaren Signalgruppe eines Substrats kann es sich z.B. um einen im sichtbaren Bereich des Spektrums bestimmbaren Farbstoff, einen Fluoreszenzfarbstoff oder einen im UV-Bereich bestimmbaren Farbstoff handeln. Bevorzugterweise werden Peptide verwendet, die an der Carboxygruppe eines Argininrests einen durch Amidbindung gebundenen Farbstoffrest aufweisen. Besonders geeignet sind hierfür p-Nitroanilidgruppen (pNA) und 5-Amino-2-Nitro-Benzoesäurederivate (ANBA), sowie von diesen durch Substitution abgeleitete Farbstoffe, welche nach Abspaltung vom Peptidanteil durch eine photometrische Messung bei 405 nm Wellenlänge quantifiziert werden können. Bevorzugterweise besteht der Peptidanteil eines spaltbaren Substrats aus 3 bis etwa 150 Aminosäureresten. In den Patentdokumenten EP 0034122 A1 und US 4,508,644 sind eine Vielzahl von geeigneten chromogenen Peptidsubstraten, deren Herstellung und deren Verwendung in gerinnungsdiagnostischen Tests beschrieben, z. B. zur Bestimmung der Gerinnungsfaktoren Faktor IIa (Thrombin) und Xa.

Die Menge des freigesetzten signalbildenden Spaltprodukts, z.B. der chromogenen, fluorogenen oder amperogenen Gruppe, verhält sich umgekehrt proportional zur Inhibitoraktivität bzw. -konzentration in der Probe. Mit Hilfe einer Kalibrationskurve, erstellt aus der Messung von Proben mit bekannten Inhibitoraktivitäten, lässt sich die Menge eines direkten Gerinnungsinhibitors in einer Patientenprobe korrekt quantifizieren.

Die amidolytische Aktivität kann kinetisch oder als Endpunktbestimmung ausgewertet werden. Bei der kinetischen Methode wird die Reaktion, d.h. die verbliebene Gerinnungsfaktoraktivität als Maß für die vorhandene Inhibitoraktivität, anhand der Umsatzrate des spaltbaren Substrats quantifiziert. Bei der Endpunktbestimmung wird nach einer vorbestimmten Messzeit die Spaltreaktion gestoppt und die Menge des freigesetzten Spaltprodukts gemessen.

Ein besonderer Vorteil des erfindungsgemäßen Verfahrens besteht darin, dass es sich lediglich durch die Vorbehandlung der Probe von einem herkömmlichen Verfahren zur Bestimmung der Heparinaktivität in einer Probe unterscheidet und daher hervorragend mit einem solchen kombiniert werden kann. So ist es z.B. möglich ein erstes Aliquot einer Probe und ein zweites Aliquot derselben Probe, welches erfindungsgemäß zunächst mit einem Oxidationsmittel und anschließend mit einem das Oxidationsmittel neutralisierenden Agens vermischt wurde, jeweils mit einem ersten Reagenz, das eine definierte Konzentration eines aktivierten Gerinnungsfaktors enthält, und einem zweiten Reagenz, das ein Gerinnungsfaktorspezifisches Substrat enthält, zu vermischen, und die Inhibitor-abhängige Inaktivierung der Aktivität des zugegebenen Gerinnungsfaktors zu bestimmen. Das Testergebnis des ersten, unbehandelten Aliquots spiegelt das gesamte antikoagulatorische Potenzial, d.h. die Summe aller Gerinnungsfaktor-inhibierenden Aktivitäten der Probe wieder, einschließlich etwaiger Heparinaktivitäten. Das Testergebnis des zweiten, erfindungsgemäß vorbehandelten Aliquots spiegelt hingegen spezifisch die Gerinnungsfaktor-inhibierenden Aktivitäten etwaiger direkter Gerinnungsfaktorinhibitoren in der Probe wieder.

Ein weiterer Aspekt der vorliegenden Enthüllung betrifft ein Testkit zur Durchführung des erfindungsgemäßen Verfahrens zur Bestimmung eines direkten Inhibitors eines Gerinnungsfaktors in einer Probe, wobei das Testkit mindestens zwei Reagenzien enthält, von denen eines ein Oxidationsmittel enthält und das andere ein das Oxidationsmittel neutralisierendes Agens. Die Reagenzien können zusätzlich Konservierungsmittel enthalten und entweder als Flüssigreagenzien oder als Lyophilisate bereitgestellt werden. Ein bevorzugtes Testkit enthält ein erstes Reagenz, das Natriumhypochlorit als Oxidationsmittel enthält und ein zweites Reagenz, das Salzsäure als neutralisierendes Agens enthält.

Ein Testkit enthält bevorzugterweise zusätzlich weitere Reagenzien, besonders bevorzugt ein Reagenz, das einen aktivierten Gerinnungsfaktor, bevorzugt Thrombin oder Faktor Xa, enthält und/oder ein Reagenz, das ein Substrat für den aktivierten Gerinnungsfaktor, welches eine nachweisbare Signalgruppe aufweist, enthält.

Die Reagenzien des erfindungsgemäßen Testkits können in flüssiger oder lyophilisierter Form bereitgestellt werden. Für den Fall, dass einige oder alle Reagenzien des Testkits als Lyophilisate vorliegen, kann das Testkit zusätzlich die zur Lösung der Lyophilisate erforderlichen Lösemittel enthalten, wie z. B. destilliertes Wasser, geeignete Puffer und/oder Standard-Human-Plasma.

### FIGURENBESCHREIBUNG

### Figur 1

Bestimmung der Rivaroxaban-spezifischen Faktor Xa-Inhibition in Heparin-haltigen Proben (siehe Beispiel 2).
Kurve 1: Rivaroxaban-Plasmen ohne Heparin (R0 bis R5), ohne erfindungsgemäße Vorbehandlung;
Kurve 2: Rivaroxaban-Plasmen mit Heparin (R0H bis R5H), ohne erfindungsgemäße Vorbehandlung; die gemessene Inhibition ist die Summe aus Rivaroxaban- und Heparin-Inhibition;
Kurve 3: Rivaroxaban-Plasmen ohne Heparin (R0 bis R5), mit erfindungsgemäßer Vorbehandlung;
Kurve 4: Rivaroxaban-Plasmen mit Heparin (R0H bis R5H), mit erfindungsgemäßer Vorbehandlung; die gemessene Inhibition ist entspricht der Rivaroxaban-Inhibition (vgl. mit Kurven 1 oder 3), die Heparin-Inhibition wurde durch die erfindungsgemäße Vorbehandlung eliminiert.

### BEISPIELE

Die folgenden Ausführungsbeispiele dienen der Veranschaulichung der Erfindung und sind nicht als Einschränkung zu verstehen.

### BEISPIEL 1

### Eliminierung der Heparinaktivität in Proben durch Zugabe eines Oxidationsmittels und eines neutralisierenden Agenzes

### Beispiel 1a)

### Bestimmung eines direkten Faktor Xa-Inhibitors in erfindungsgemäß vorbehandelten Proben

Humanem Normalplasma wurden verschiedene Mengen direkter Gerinnungsfaktor-Inhibitoren (Rivaroxaban als direkter Faktor Xa-Inhibitor, Argatroban als direkter Thrombininhibitor) oder indirekter Gerinnungsfaktor-Inhibitoren (Pentasaccharid Fondaparinux [Arixtra®], hochmolekulares Heparin [HMWH]) zugegeben.

20 µl Plasmaprobe wurden mit 10 µl einer 0,1 prozentigen hypochlorigen Säure (Natriumhypochlorit) als Oxidationsmittel vermischt, und der Reaktionsansatz wurde bei +37°C für 30 Sekunden inkubiert. Anschließend wurden dem Reaktionsansatz 10 µl 0,1 prozentige Salzsäurelösung zur Neutralisierung des Oxidationsmittels zugegeben, und der Reaktionsansatz wurde bei +37°C für 30 Sekunden inkubiert. Anschließend wurden dem Reaktionsansatz 95 µl Faktor Xa-Reagenz (humaner Faktor Xa, 1 U/mL in TRIS Puffer, pH 8.0) zugegeben, und der Reaktionsansatz wurde bei +37°C für 30 Sekunden inkubiert. Anschließend wurden dem Reaktionsansatz 80 µl eines chromogenen Faktor Xa-Substrats (Z-D-Leu-Gly-Arg-ANBAmethylamid, 4 mmol/L) zugegeben, und die Umsetzung des Substrats (in ΔE/Zeit) wurde bei einer Wellenlänge von 405 nm in einem automatischen Gerinnungsmessgerät (BCS® System, Siemens Healthcare Diagnostics) ermittelt.

Die Verringerung des Substratumsatzes im Vergleich zu Plasma ohne jeglichen Inhibitor korreliert mit der Menge an Inhibitor in der Probe.

Die Ergebnisse sind in Tabelle 1 dargestellt. Die erfindungsgemäße Vorbehandlung der Proben bewirkt eine fast vollständige Eliminierung der Hemmung von Faktor Xa durch indirekte Inhibitoren wie Arixtra (Fondaparinux) oder hochmolekulares Heparin. Die Faktor Xa-inhibierende Wirkung von direkten Inhibitoren wie Rivaroxaban wird durch die erfindungsgemäße Vorbehandlung der Proben jedoch nicht beeinträchtigt. In Proben, die einen direkten Thrombininhibitor (Argatroban) enthalten, findet ohnehin keine Faktor Xa-Inhibition statt.

**Tabelle 1**

| **Plasma** | | **ΔE/min** | **F Xa-Inhibition** |
|---|---|---|---|
| **ohne Inhibitor** | | 2268 | **0 %** |
| **mit direktem Inhibitor** | 200 ng/mL Rivaroxaban | 1296 | **43 %** |
| | 20 µg/mL Argatroban | 2176 | **4 %** |
| **mit indirektem Inhibitor** | 1 µg/mL Arixtra | 2319 | **4 %** |
| | 4 µg/mL Arixtra | 2344 | **3** % |
| | 0,5 U/mL Heparin | 2353 | **4 %** |
| | 2,0 U/mL Heparin | 2283 | **1 %** |

### Beispiel 1b)

### Bestimmung eines direkten Thrombin-Inhibitors in erfindungsgemäß vorbehandelten Proben

20 µl Plasmaprobe wurden mit 10 µl einer 0,1 prozentigen hypochlorigen Säure (Natriumhypochlorit) als Oxidationsmittel vermischt, und der Reaktionsansatz wurde bei +37°C für 30 Sekunden inkubiert. Anschließend wurden dem Reaktionsansatz 10 µl 0,1 prozentige Salzsäurelösung zur Neutralisierung des Oxidationsmittels zugegeben, und der Reaktionsansatz wurde bei +37°C für 30 Sekunden inkubiert. Anschließend wurden dem Reaktionsansatz 140 µl Thrombin-Reagenz (Rinderthrombin, 6 U/mL) zugegeben, und der Reaktionsansatz wurde bei +37°C für 30 Sekunden inkubiert. Anschließend wurden dem Reaktionsansatz 50 µl eines chromogenen Thrombin-Substrats (Tos-L-Gly-Pro-Arg-ANBA-isopropylamid, 4 mmol/L) zugegeben, und die Umsetzung des Substrats (in ΔE/Zeit) wurde bei einer Wellenlänge von 405 nm in einem automatischen Gerinnungsmessgerät (BCS® System, Siemens Healthcare Diagnostics) ermittelt.

Die Verringerung des Substratumsatzes im Vergleich zu Plasma ohne jeglichen Inhibitor korreliert mit der Menge an Inhibitor in der Probe.

Die Ergebnisse sind in Tabelle 2 dargestellt. Die erfindungsgemäße Vorbehandlung der Proben bewirkt eine fast vollständige Eliminierung der Hemmung von Thrombin durch indirekte Inhibitoren wie Arixtra (Fondaparinux) oder hochmolekulares Heparin. Die Thrombin-inhibierende Wirkung von direkten Inhibitoren wie Argatroban wird durch die erfindungsgemäße Vorbehandlung der Proben jedoch nicht beeinträchtigt. In Proben, die einen direkten Faktor Xa-Inhibitor (Rivaroxaban) enthalten, findet ohnehin keine Thrombin-Inhibition statt.

**Tabelle 2**

| **Plasma** | | **ΔE/min** | **Thrombin-Inhibition** |
|---|---|---|---|
| **ohne Inhibitor** | | 2623 | **0 %** |
| **mit direktem Inhibitor** | 200 ng/mL Rivaroxaban | 2612 | **0 %** |
| | 20 µg/mL Argatroban | 140 | **95 %** |
| | 200 µg/mL Argatroban | 36 | **99 %** |
| **mit indirektem Inhibitor** | 1 µg/mL Arixtra | 2480 | **5 %** |
| | 4 g/mL Arixtra | 2579 | **2 %** |
| | 0,5 U/mL Heparin (HMW) | 2533 | **3 %** |
| | 2,0 U/mL Heparin (HMW) | 2594 | **1 %** |

### BEISPIEL 2

### Spezifische Bestimmung des direkten Faktor Xa-Inhibitors Rivaroxaban in Heparin-haltigen Proben

Humanem Normalplasma wurden verschiedene Mengen des direkten Faktor Xa-Inhibitors Rivaroxaban bzw. verschiedene Mengen des direkten Faktor Xa-Inhibitors Rivaroxaban und verschiedene Mengen des indirekten Faktor Xa-Inhibitors Heparin (HMWH) zugegeben (siehe Tabelle 3).

**Tabelle 3**

| **Probe** | R0 | R1 | R2 | R3 | R4 | R5 | R0H | R1H | R2H | R3H | R4H | R5H |
|---|---|---|---|---|---|---|---|---|---|---|---|---|
| Rivaroxaban (ng/mL) | 0 | 100 | 200 | 300 | 400 | 500 | 0 | 100 | 200 | 300 | 400 | 500 |
| Heparin (U/ml) | 0 | 0 | 0 | 0 | 0 | 0 | 0, 4 | 0, 4 | 0, 4 | 0, 4 | 0, 4 | 0, 4 |

9 µl Plasmaprobe wurden zunächst mit 6 µl Wasser und dann mit 8 µl einer 0,1 prozentigen hypochlorigen Säure (Natriumhypochlorit) als Oxidationsmittel vermischt und auf +37°C temperiert. Anschließend wurden dem Reaktionsansatz 8 µl 0,1 prozentige Salzsäurelösung zur Neutralisierung des Oxidationsmittels zugegeben, und der Reaktionsansatz wurde bei +37°C für 120 Sekunden inkubiert. Anschließend wurden dem Reaktionsansatz 150 µl Faktor Xa-Reagenz (humaner Faktor Xa, 1 U/mL in TRIS Puffer, pH 8.0) zugegeben, und der Reaktionsansatz wurde bei +37°C für 120 Sekunden inkubiert. Anschließend wurden dem Reaktionsansatz 30 µl eines chromogenen Faktor Xa-Substrats (Z-D-Leu-Gly-Arg-ANBAmethylamid, 4 mmol/L) zugegeben, und die Umsetzung des Substrats (in ΔE/Zeit) wurde bei einer Wellenlänge von 405 nm in einem automatischen Gerinnungsmessgerät (BCS® System, Siemens Healthcare Diagnostics) ermittelt.

Parallel wurden die Proben ohne die erfindungsgemäße Vorbehandlung mit 0,1 prozentiger hypochloriger Säure und mit 0,1 prozentiger Salzsäurelösung mit dem vorbeschriebenen Verfahren untersucht.

Die Verringerung des Substratumsatzes im Vergleich zu Plasma ohne jeglichen Inhibitor korreliert mit der Menge an Inhibitor in der Probe.

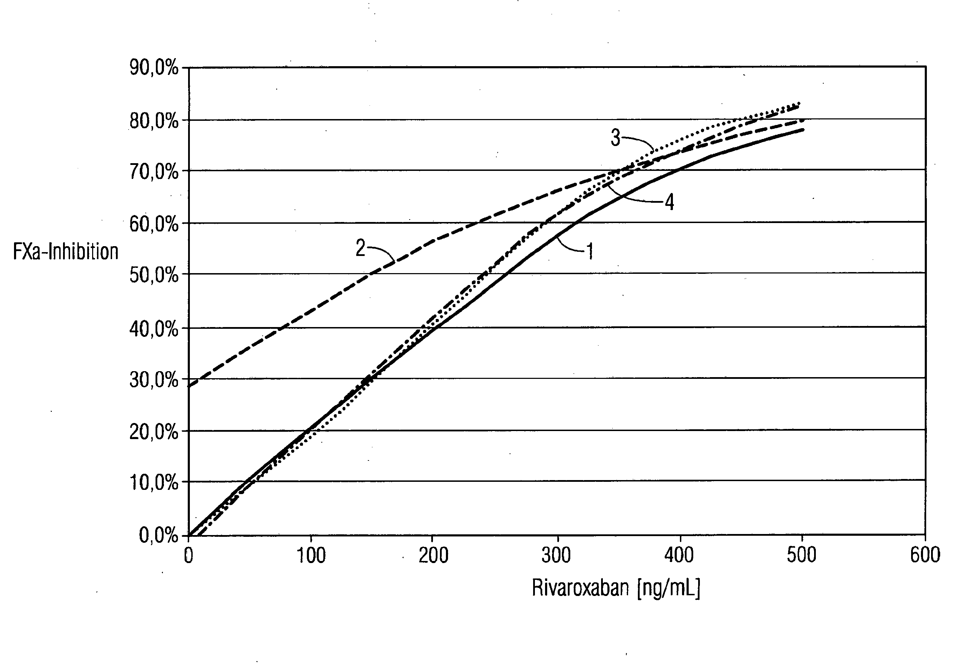

Die Ergebnisse sind in Figur 1 dargestellt. Die erfindungsgemäße Vorbehandlung von Proben, die Rivaroxaban und Heparin enthalten , hat den Effekt, dass nur noch eine Faktor Xa-Inhibition gemessen wird, die der Faktor Xa-Inhibition von Rivaroxaban entspricht (vergleiche Kurve 2, ohne Vorbehandlung und Kurve 4, mit Vorbehandlung). Das erfindungsgemäße Verfahren ermöglicht daher die spezifische Bestimmung der Rivaroxaban-Aktivität in Heparin-haltigen Proben.

## Patentansprüche

1. Verfahren zur Bestimmung eines direkten Inhibitors eines Gerinnungsfaktors in einer Probe, wobei der direkte Inhibitor des Gerinnungsfaktors ausgewählt ist aus der Gruppe der direkten Thrombininhibitoren Hirudin, Dabigatran, Melagatran, Argatroban, Ximelagatran, Bivalirudin, Lepirudin, MCC-977,SSR-182289, TGN-255, TGN-167, ARC-183 und Odiparcil oder aus der Gruppe der direkten Faktor Xa-Ihhibitoren Rivaroxaban, Apixaban, Otamixaban, LY 517717, YM 153, DU-176b, DX-9065a und KFA-19982, wobei das Verfahren folgende Schritte in der genannten Reihenfolge aufweist:
a) Vermischen der Probe mit einem Oxidationsmittel zu einem Reaktionsansatz;
b) Inkubation des Reaktionsansatzes;
c) Vermischen des Reaktionsansatzes mit einem das Oxidationsmittel neutralisierenden Agens;
d) Zugabe einer definierten Menge des aktivierten Gerinnungsfaktors Thrombin oder Faktor Xa zum Reaktionsansatz;
e) Bestimmung der Hemmung des zugesetzten aktivierten Gerinnungsfaktors.

2. Verfahren nach Anspruch 1, wobei in Schritt a) die Probe mit einem Oxidationsmittel aus der Gruppe Hypochloride und deren Salze, insbesondere Natrium- oder Kaliumhypochlorit, reaktive Halogene, Wasserstoffperoxide, Peroxomonoschwefelsäure, Peroxodischwefelsäure, Chloramin B, Chloramin T, Hypochlorsäure, N-Chlorosuccinemid oder deren Salze vermischt wird.

3. Verfahren nach einem der vorhergehenden Ansprüche, wobei in Schritt c) der Reaktionsansatz mit einem neutralisierenden Agens aus der Gruppe Halogensäuren, bevorzugt niedermolare, wässrige Lösungen von Chlorwasserstoffsäuren, besonders bevorzugt Salzsäure; Fruchtzucker; Polyol; Fruchtsäuren und deren Salze vermischt wird.

4. Verfahren nach einem der vorhergehenden Ansprüche, wobei die Inkubationsdauer in Schritt b) mindestens 30 Sekunden beträgt, bevorzugterweise 30 bis 180 Sekunden, besonders bevorzugt 90 bis 120 Sekunden.

5. Verfahren nach einem der vorhergehenden Ansprüche, wobei nach dem Schritt c) dem Reaktionsansatz ferner ein chromogenes, fluorogenes oder anderweitig markiertes Substrat, welches von dem aktivierten Gerinnungsfaktor Thrombin oder Faktor Xa spezifisch gespalten wird, zugegeben wird und die Menge des freigesetzten signalbildenden Spaltprodukts gemessen wird.

## Claims

1. Method for determining a direct inhibitor of a coagulation factor in a sample, wherein the direct inhibitor of the coagulation factor is selected from the group of the direct thrombin inhibitors hirudin, dabigatran, melagatran, argatroban, ximelagatran, bivalirudin, lepirudin, MCC-977, SSR-182289, TGN-255, TGN-167, ARC-183 and odiparcil or from the group of the direct factor Xa inhibitors rivaroxaban, apixaban, otamixaban, LY 517717, YM 153, DU-176b, DX-9065a and KFA-1982, wherein the method comprises the following steps in the order stated:
a) mixing the sample with an oxidizing agent to form a reaction mix;
b) incubating the reaction mix;
c) mixing the reaction mix with an agent which neutralizes the oxidizing agent;
d) adding a defined amount of the activated coagulation factor thrombin or factor Xa to the reaction mix;
e) determining the inhibition of the activated coagulation factor added.

2. Method according to Claim 1, wherein, in step a), the sample is mixed with an oxidizing agent from the group consisting of hypochlorites and salts thereof, in particular sodium or potassium hypochlorite, reactive halogens, hydrogen peroxides, peroxymonosulfuric acid, peroxydisulfuric acid, chloramine B, chloramine T, hypochlorous acid, N-chlorosuccinimide or salts thereof.

3. Method according to any of the preceding claims, wherein, in step c), the reaction mix is mixed with a neutralizing agent from the group consisting of halogen acids, preferably low-molar aqueous solutions of chlorine acids, particularly preferably hydrochloric acid; fructose; polyols; fruit acids and salts thereof.

4. Method according to any of the preceding claims, wherein the duration of incubation in step b) is at least 30 seconds, preferably 30 to 180 seconds, particularly preferably 90 to 120 seconds.

5. Method according to any of the preceding claims, wherein, after step c), a chromogenic, fluorogenic or differently labeled substrate which is specifically cleaved by the activated coagulation factor thrombin or factor Xa is also added to the reaction mix and the amount of signal-producing cleavage product released is measured.

## Revendications

1. Procédé de détermination d'un inhibiteur direct d'un facteur de coagulation dans un échantillon, l'inhibiteur direct du facteur de coagulation étant choisi dans le groupe des inhibiteurs directs de thrombine hirudine, dabigatran, mélagatran, argatroban, ximélagatran, bivalirudine, lépirudine, MCC-977, SSR-182289, TGN-255, TGN-167, ARC-183 et odiparcil ou dans le groupe des inhibiteurs directs du facteur Xa rivaroxaban, apixaban, otamixaban, LY 517717, YM 153, DU-176b, DX-9065a et KFA-1982, le procédé ayant les stades suivants dans l'ordre mentionné :
a) mélange de l'échantillon à un agent d'oxydation pour obtenir une masse réactionnelle ;
b) incubation de la masse réactionnelle ;
c) mélange de la masse réactionnelle à un agent neutralisant l'agent d'oxydation ;
d) addition d'une quantité définie du facteur de coagulation activé thrombine ou facteur Xa à la masse réactionnelle ;
e) détermination de l'inhibition du facteur de coagulation activé qui a été ajouté.

2. Procédé suivant la revendication 1, dans lequel, dans le stade a), on mélange l'échantillon à un agent d'oxydation du groupe des chlorhydrates et de leurs sels, notamment de l'hypochlorite de sodium ou de l'hypochlorite de potassium, des halogènes réactifs, du peroxyde d'hydrogène, de l'acide peroxomonosulfurique, de l'acide peroxodisulfurique, de la chloramine B, de la chloramine T, de l'acide hypochlorique, du N-chlorosuccinimide ou de leurs sels.

3. Procédé suivant l'une des revendications précédentes, dans lequel, dans le stade c), on mélange la masse réactionnelle à un agent neutralisant du groupe des acides halogénés, de préférence de solutions aqueuses d'acides chlorhydriques, notamment de préférence d'acide chlorhydrique ; de sucres de fruit ; de polyols ; d'acides de fruit et de leurs sels.

4. Procédé suivant l'une des revendications précédentes, dans lequel la durée d'incubation, dans le stade b), est d'au moins 30 secondes, de préférence de 30 à 180 secondes, d'une manière particulièrement préférée de 90 à 120 secondes.

5. Procédé suivant l'une des revendications précédentes, dans lequel, après le stade c), on ajoute, à la masse réactionnelle en outre, un substrat chromogène, fluorogène ou marqué d'une autre façon, qui est décomposé spécifiquement par le facteur de coagulation activé thrombine ou facteur Xa, et on mesure la quantité de produit de décomposition libéré qui forme un signal.
